# EUROPEAN PATENT APPLICATION

(11) **EP 4 105 227 A1**
(43) Date of publication of application: **21.12.2022**
(21) Application number: 21754618.3
(22) Date of filing: 10.02.2021
(51) Int. Cl.: C07K 7/08, C12N 15/11, C07K 19/00, A61K 38/10, A61K 45/06, A61K 39/395, A61P 35/00, A61P 29/00

(54) **IMMUNE CHECKPOINT TIM3-TARGETING BINDING PEPTIDE AND APPLICATION THEREOF**

(30) Priority: 11.02.2020 CN 202010086367
(71) Applicant: Sparx Biotech (Jiangsu) Co., Ltd., Jiangsu 225001 (CN)
(72) Inventor: SHOU, Chengchao, Beijing 100142 (CN); ZHAO, Chuanke, Beijing 100142 (CN); ZHONG, Tangwu, Beijing 100142 (CN); WANG, Shuntao, Beijing 100142 (CN)
(74) Representative: Brand Murray Fuller LLP
(86) International application number: PCT/CN2021/076421
(87) International publication number: WO 2021/160146

(57) **Abstract**

The present invention provides an immune checkpoint TIM3-targeting binding peptide and application thereof. The present invention first provides a polypeptide (a) or (b) as follows or a pharmaceutically acceptable salt thereof: (a) a polypeptide consisting of the amino acid sequence as set forth in any one of SEQ ID No.1 to SEQ ID No. 16; (b) a derived polypeptide which has the same function as (a) and is obtained by substituting, adding and/or deleting one or more amino acids in the amino acid sequence as set forth in any one of SEQ ID No.1 to SEQ ID No. 16. The polypeptide can specifically bind to TIM3, and has an application prospect in the fields of regulation/improvement of T cell immunocompetence and anti-tumor treatment.

## Description

### TECHNICAL FIELD

The invention relates to an immune checkpoint TIM3-targeting binding peptide and use thereof, and in particular, to a polypeptide that can specifically bind to TIM3 and its applications in the fields of regulation/improvement of T cell immunocompetence and anti-tumor treatment. The application relates to the biopharmaceutical (peptide drugs) and genetic engineering fields.

### BACKGROUND OF INVENTION

Tumor immunotherapy has become the fourth effective therapy for tumors following surgery, chemotherapy, and radiotherapy. Recently, inhibitors of immune checkpoints represented by PD-1 and PD-L1 (programmed death receptor 1 and its ligand) and CTLA4 (cytotoxic T lymphocyte-associated antigen 4) have become study hotspots of clinical tumor immunotherapy. A variety of immune checkpoint inhibitors have been approved and marketed in several countries, and have gradually become one of the most desirable therapeutic approaches in the field of tumors (Datar, I.; Sanmamed, M. F.; Wang, J.; Henick, B. S.; Choi, J.; Badri, T.; Dong, W.; Mani, N.; Toki, M.; Mejías, L. D.; Lozano, M. D.; Perez-Gracia, J. L.; Velcheti, V; Hellmann, M. D.; Gainor, J. F.; McEachern, K.; Jenkins, D.; Syrigos, K.; Politi, K.; Gettinger, S.; Rimm, D. L.; Herbst, R. S.; Melero, I.; Chen, L.; Schalper, K. A., Expression Analysis and Significance of PD-1, LAG-3, and TIM-3 in Human Non-Small Cell Lung Cancer Using Spatially Resolved and Multiparametric Single-Cell Analysis. Clinical cancer research: an official journal of the American Association for Cancer Research 2019, 25 (15), 4663-4673). The study on the immune checkpoints, represented by PD-1, is currently producing significant fundamental and clinical breakthroughs worldwide. Related antibody drugs have been approved for clinical tumor therapy. Next, TIM3, LAG3, BTLA, VISTA, etc., are research hotspots in the current anti-tumor immune checkpoint (Das, M.; Zhu, C.; Kuchroo, V. K., Tim-3 and its role in regulating anti-tumor immunity. Immunol Rev 2017, 276 (1), 97-111). However, it has been shown recently that upon being resistant to clinical anti-PD-1 therapy, a secondary increase in the expression of TIM3 was occurred, which promoted T cell failure, thereby leading to immune escape. The combined blockade therapy of TIM3 and PD-1 has shown good efficacy in the treatment of diseases such as malignant melanoma and non-small cell lung cancer, and can significantly inhibit tumor growth and prolong survival (He, Y; Cao, J.; Zhao, C.; Li, X.; Zhou, C.; Hirsch, F. R., TIM-3, a promising target for cancer immunotherapy. OncoTargets and therapy 2018, 11, 7005-7009; Romero, D., Immunotherapy: PD-1 says goodbye, TIM-3 says hello. Nature reviews. Clinical oncology 2016, 13 (4), 202-3). Furthermore, targeted inhibition of TIM3 alone or synergic combination with blockade therapies such as PD-1/PD-L1 can effectively reverse the progression of T lymphocyte failure, enhance anti-tumor immune response, and inhibit tumor growth.

The *TIM* gene family was first discovered in 2001 during the analysis of genes susceptible to asthma. Its members include *TIM1~TIM8* and the human TIM gene family includes *TIM-1, TIM-3* and *TIM-4.* TIM3 (T lymphocyte immunoglobulin mucin-3) is a type I membrane protein consisting of 301 amino acids (murine TIM3 is 281 amino acids in length) (Monney, L.; Sabatos, C. A.; Gaglia, J. L.; Ryu, A.; Waldner, H.; Chernova, T.; Manning, S.; Greenfield, E. A.; Coyle, A. J.; Sobel, R. A.; Freeman, G. J.; Kuchroo, V. K., Th1-specific cell surface protein Tim-3 regulates macrophage activation and severity of an autoimmune disease. Nature 2002, 415 (6871), 536-41), a class of T cell surface inhibitory molecules, and are expressed primarily on a variety of immune cells such as T cells, monocytes, macrophages, and dendritic cells, as well as on a variety of tumor cells. Its main ligands include Galectin-9, high mobility family protein-1 (HMGB1), carcinoembryonic antigen-associated adhesion molecule-1 (CEACAM1), phosphatidylserine (PS). However, the mechanisms underlying the interaction of TIM3 with its ligands for immune suppression are still not well known (He, Y; Cao, J.; Zhao, C.; Li, X.; Zhou, C.; Hirsch, F. R., TIM-3, a promising target for cancer immunotherapy. OncoTargets and therapy 2018, 11, 7005-7009). The immunosuppressive regulation to TIM3 involves that the key tyrosine residues in TIM3, after being phosphorylated by tyrosine kinase family members such as LCK and Fyn, interact with proteins containing the SH2 domain, resulting in activation of NFAT/AP-1 and NF-κB, ultimately leading to activation of T cells and accelerated T cell depletion. In addition, TIM3 promotes proliferation of large number of Treg cells and MDSC, and impedes activation and differentiation of DCs, thereby inhibiting the anti-tumor immunity of an organism (Tao Jinglian, Li Lijuan, and Shao Zonghong, Study on progress in the role of TIM3 in tumor microenvironment [J]. Chinese Journal of Immunology, 2016, 32 (07) : 1070-1073).

### SUMMARY OF INVENTION

It is an object of the application to provide a polypeptide that can specifically bind to TIM3.

Another object of the application is to provide the use of the polypeptides.

The inventors have obtained a group of antagonist molecules of TIM3 polypeptide with good potential in anti-tumor application via experiments, which have the amino acid sequence as set forth in any one of SEQ ID No.1 to SEQ ID No.16. Experimental studies by the inventors have shown that, these polypeptides can not only specifically bind to TIM3 at cellular and protein levels, but also block interaction between TIM3/Galectin-9 and compete with Galectin-9 for binding to TIM3 in terms of affinity. These polypeptides may activate CD4+ T cells, promote upregulation of expression of related cytokines (IFN-γ and IL-2), exhibit good synergistic effect in combination with PD-1 inhibitors (Opdivo) and reverse immunosuppression caused by TIM3-Galectin-9 interaction in terms of biological function and activity. TIM3-binding peptides can inhibit tumor growth and have synergistic inhibitory effects on tumor growth in combination with the PD-L1 inhibitor atezolizum in terms of anti-tumor *in vivo.* These results indicate that the immune checkpoint TIM3-targeting binding peptide according to the invention, whether used alone or in combination with other immune checkpoint inhibitors, have potential application prospects of clinically inhibiting TIM3 signaling-related tumors and inflammatory diseases.

Thus, in one aspect, the application provides a polypeptide of (a) or (b) or a pharmaceutically acceptable salt thereof:
(a) a polypeptide consisting of the amino acid sequence as set forth in any one of SEQ ID No.1 to SEQ ID No. 16;
(b) a derived polypeptide which is obtained by substituting, adding and/or deleting one or more amino acids in the amino acid sequence as set forth in any one of SEQ ID No.1 to SEQ ID No. 16 and has the same function as (a).

The polypeptides consisting of the amino acid sequence as set forth in any one of SEQ ID No.1 to SEQ ID No. 16 according to the invention are capable of specifically binding to TIM3, and are also referred to herein as TIM3-targeting binding peptides. Specifically, TIM3-targeting binding peptides according to the invention include 12-peptides and 20-peptides targeting TIM3 having the specific amino acid sequences as follows:
the amino acid sequences of the 12-peptides (targeting TIM3):
   P29-ADFRVMADMSMY (SEQ ID No. 1)
   P1309-SHTTTEHAKDKI (SEQ ID No. 2)
   P26-GLIPLTTMHIGK (SEQ ID No. 3)
   P1310-TFLNSVPTYSYW (SEQ ID No. 4)
   P1307-SHDTRSPFTWGR (SEQ ID No. 5)
   P24-GSWNTFRAQPTI (SEQ ID No. 6)
   P22-SFAGVHQFRPAP (SEQ ID No. 7)
   P1303-ALWPSSSMSSTI (SEQ ID No. 8)
   P130-HDAFPRPHLFRN (SEQ ID No. 9)
   P20-LTPHKHHKHLHA (SEQ ID No. 10)
the amino acid sequences of the 20-peptides (targeting TIM3):
   K1- HYEADPYEAWYDAWDDIHTY (SEQ ID No. 11)
   K2- FTYAHPVSHRWESSVHVDQW (SEQ ID No. 12)
   K3- SQEVFAWTFSPRWTTAPKAL (SEQ ID No. 13)
   K5- WQQNNPHLWDVFLGVPRTTE (SEQ ID No. 14)
   K7- QNPPLENTVYQEPFWQHAVD (SEQ ID No. 15)
   K10- SPFYYSGHQWVPHPRQAASY (SEQ ID No. 16).

In another aspect, the application also provides the use of the polypeptides (e.g., the polypeptides of (a) or (b), i.e., TIM3-targeting binding peptides according to the invention) according to the invention as binding peptides targeting the immune checkpoint TIM3. TIM3-targeting binding peptides according to the invention have the ability to specifically bind to TIM3, including specific affinity for TIM3 at the protein and cellular levels. TIM3-targeting binding peptides according to the invention have a good ability to compete with Galectin-9 for binding to TIM3, including specific affinity for TIM3 at the protein and cellular levels.

In another aspect, the application also provides a polynucleotide encoding the polypeptides according to the invention. A person skilled in the art would be able to design corresponding nucleotide sequences based on the amino acid sequence as set forth in any one of SEQ ID No.1 to SEQ ID No. 16. The polynucleotides may be inserted into an expression vector or a host cell for expressing the polypeptides.

In another aspect, the application also provides a fusion protein comprising the amino acid sequences of the polypeptides according to the invention. The polypeptides consisting of the amino acid sequence as set forth in any one of SEQ ID No.1 to SEQ ID No. 16 or the derivative polypeptides having the same function, can be fused with other peptides or proteins to form a fusion protein by a person skilled in the art as needed.

In another aspect, the application also provides a pharmaceutical composition comprising a polypeptide according to the invention, or a pharmaceutically acceptable salt thereof, or a polynucleotide encoding the polypeptide according to the invention, or a fusion protein comprising the amino acid sequences of the polypeptides according to the invention; and a pharmaceutically acceptable carrier or excipient.

According to a particular embodiment of the application, the pharmaceutical composition according to the invention further comprises a PD-1 inhibitor or a PD-L1 inhibitor.

According to a particular embodiment of the invention, in the pharmaceutical composition according to the invention, the PD-1 inhibitor is Opdivo; the PD-L1 inhibitor is atezolizum. In particular, TIM3-targeting binding peptides according to the invention have a synergistic effect in combination with the PD-1 inhibitor (Opdivo) for the intervention and modulation of T cell activity.

In another aspect, the application also provides the use of the polypeptide, the polynucleotide, the fusion protein or the pharmaceutical composition according to the invention in the manufacture of a reagent that enhances T cell activity.

In another aspect, the application also provides the use of the polypeptide, the polynucleotide, the fusion protein, or the pharmaceutical composition according to the invention in the manufacture of a reagent for the promotion of elevated expression of the immune factors IFN-γ and/or IL-2 secreted by T cells.

The activity of the polypeptides according to the invention is primarily embodied in the use in modulating T cell activity. TIM3-targeting binding peptides according to the invention have the ability to activate T cells and enhance the expression levels of the cytokines IFN-γ and/or IL-2.

In another aspect, the application also provides the use of the polypeptide, the polynucleotide, the fusion protein, or the pharmaceutical composition according to the invention in the manufacture of a reagent that has the ability to reverse Galectin-9 mediated inhibition of T cell activation.

In another aspect, the application also provides the use of the polypeptide, the polynucleotide, the fusion protein or the pharmaceutical composition according to the invention in the manufacture of a medicament for inhibiting tumor growth. TIM3-targeting binding peptides according to the invention can inhibit tumor growth, and can have a synergistic effect of inhibiting tumor growth in combination with the PD-L1 inhibitor atezolizum. In other words, the application also provides an anti-tumor method comprising administering to a subject an effective amount of the polypeptide, the polynucleotide, the fusion protein or the pharmaceutical composition according to the invention, wherein the polypeptide is used to binds to TIM3 to inhibit tumor growth. In particular, the administration route may be, for example, intravenous infusion or the like.

In another aspect, the application also provides the use of the polypeptide, the polynucleotide, the fusion protein, or the pharmaceutical composition according to the invention in the manufacture of a medicament for the treatment of relevant tumors and/or inflammatory diseases by inhibition of TIM3 signaling.

It will be appreciated by those skilled in the art that the polypeptides according to the invention may be applied in any forms to prepare the formulations or medicaments, including, but not limited to, polypeptides or pharmaceutically acceptable salts thereof, polynucleotides encoding the polypeptides according to the invention or fusion products thereof with other polynucleotides, vectors or host cells containing the polynucleotides, fusion proteins containing the amino acid sequence of the polypeptides according to the invention (fusion products of the polypeptides according to the invention with other polypeptides or proteins), and the like.

In summary, whether used alone or in combination with other immune checkpoint inhibitors, the polypeptides have a potential application prospect of clinically treating relevant tumors and inflammatory diseases via inhibition of TIM3 signaling.

### BRIEF DESCRIPTION OF THE DRAWINGS

FIG. 1 shows Western Blot detection to identify stable expression of recombinant human TIM3 in 293T cells.
FIG. 2 shows phage-positive clones bound to 293T-TIM3, which were enriched and recovered by phage 12 peptide library subtraction screening in each round.
FIG. 3 shows cell ELISA comparison and analysis of the ratio of OD490 values of positive clones bound to target cells (293T-TIM3) to those bound to control cells (293T).
FIG. 4 shows the 12-peptides (targeting TIM3) occurred at a higher frequency via NGS sequencing and their amino acid sequences.
FIG. 5 shows a synthesis report of preferred 10 12-peptides (targeting TIM3).
FIG. 6 shows the amino acid sequences and related physicochemical properties of preferred 6 20-peptides (targeting TIM3).
FIG. 7 shows the affinity (comprising KD values) of K2-20-peptide (targeting TIM3) to TIM3 via SPR detection.
FIG. 8 shows the affinity (comprising KD values) of K3-20-peptide (targeting TIM3) to TIM3 via SPR detection.
FIG. 9 shows the affinity (comprising KD values) of K5-20-peptide (targeting TIM3) to TIM3 via SPR detection.
FIG. 10 shows the affinity (comprising KD values) of K10-20-peptide (targeting TIM3) to TIM3 via SPR detection.
FIG. 11 shows the affinity (comprising KD values) of P20-12-peptide (targeting TIM3) to TIM3 via SPR detection.
FIG. 12 shows the affinity (comprising KD values) of P24-12-peptide (targeting TIM3) to TIM3 via SPR detection.
FIG. 13 shows the affinity (comprising KD values) of P29-12-peptide (targeting TIM3) to TIM3 via SPR detection.
FIG. 14 shows the affinity (comprising KD values) of P1301-12-peptide (targeting TIM3) to TIM3 via SPR detection.
FIG. 15 shows the affinity (comprising KD values) of P1303-12-peptide (targeting TIM3) to TIM3 via SPR detection.
FIG. 16 shows the affinity (comprising KD values) of P1307-12-peptide (targeting TIM3) to TIM3 via SPR detection.
FIG. 17 shows the identification of the biological activity (INF-γ levels expressed by activated T cells) of the 20-peptides (targeting TIM3) K1, K2, K3, K5, K7, K10 of the MLR system detected by ELISA.
FIG. 18 shows the identification of the biological activity (INF-γ levels expressed by activated T cells) of 12-peptides (targeting TIM3) P20, P22, P24, P26, P29, P1301, P1303, P1307, P1309, P1310 of the MLR system detected by ELISA.

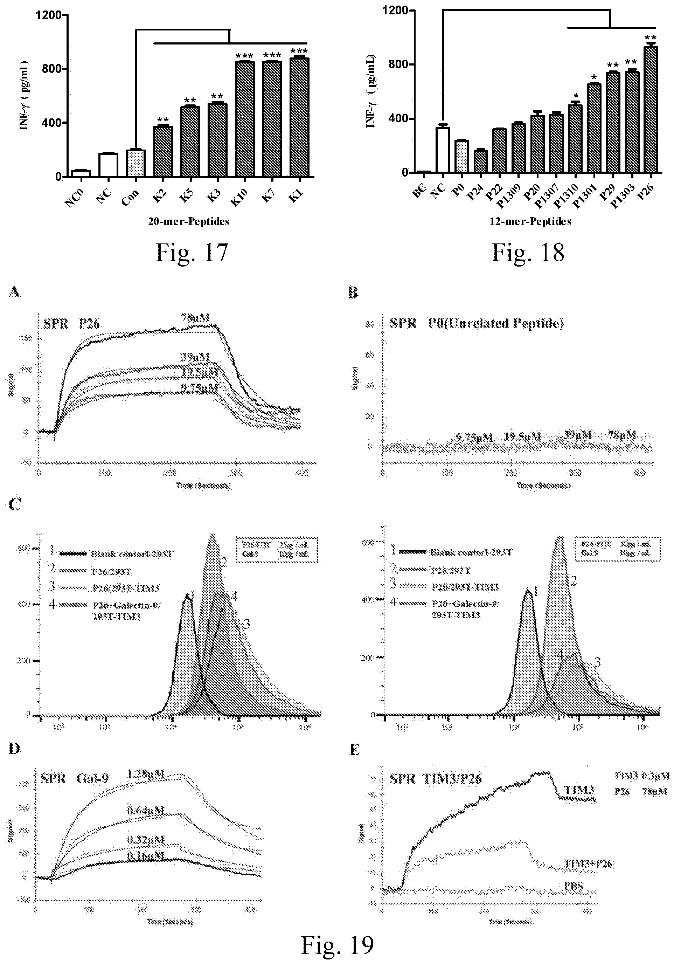

FIG. 19 shows the binding affinity of P26-12-peptide (targeting TIM3) to TIM3, in which Panel A: the KD value of P26 bound to TIM3 detected by SPR (a TIM3 sensor chip); Panel B: the binding curve of the negative control peptide P0 to TIM3 detected by SPR; Panel C: the KD value of Galectin-9 (a ligand of TIM3) bound to TIM3 detected by SPR (a TIM3 sensor chip); Panel D: the experiment of P26 competing with the ligand Galectin-9 for binding to TIM3 detected by SPR (a Galectin-9 sensor chip) (difference in binding signals between TIM3 and Galectin-9 vs. the mixture of TIM3 and P26 and Galectin-9) was detected by SPR; and Panel E: the binding specificity of different concentrations of P26-FITC to 293T-TIM3 cells and the ability of P26-FITC competing with Galectin-9 for binding to 293T-TIM3 cells examined by flow cytometry.

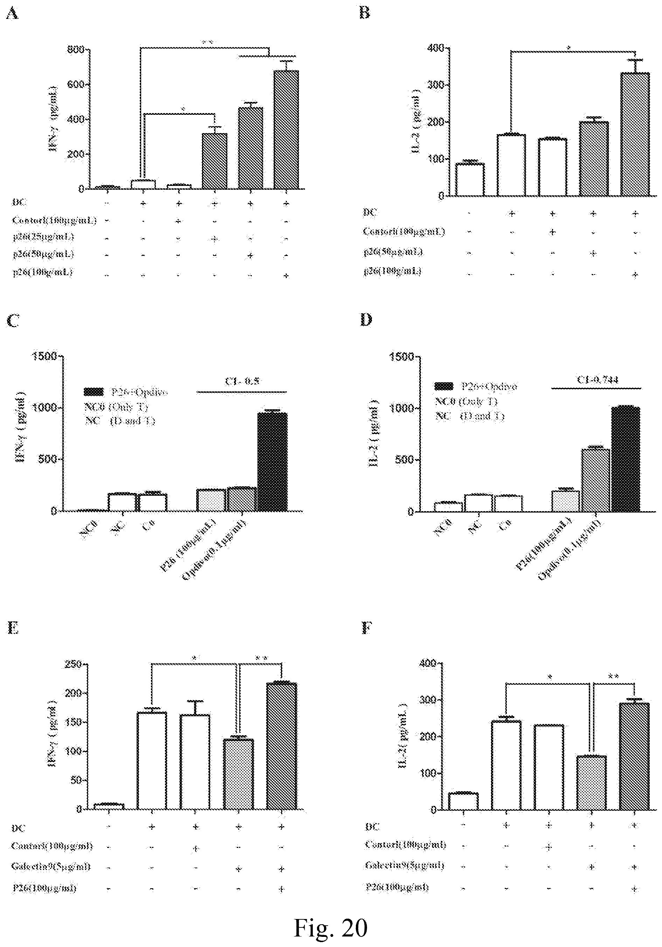

FIG. 20 shows the functional activity experiment of P26-12-peptide (targeting TIM3) and its combinations with other drugs in activation of CD4+T cells (MLR system, ELISA assay). Panels A and B show the levels of IFN-γ and IL-2 secreted by CD4+ T cells activated by different concentrations of P26. Panels C and D illustrate a synergistic experiment of P26 and Opdivo (a PD-1 inhibitor), and show the levels of IFN-γ and IL-2 secreted by CD4+ T cells activated by P26 (100 µg/mL), Opdivo (0.1 µg/mL), the combination of P26 (100µg/mL) and Opdivo (0.1 µg/mL). Panels E and F illustrate a competition inhibition experiment of P26 and the ligand Galectin-9, and show that P26 (100 µg/mL) reversed Galectin-9 (5 µg/mL)-mediated inhibition of CD4+ T cell activation (increased expression levels of IFN-γ and IL-2).
FIGs. 21 and 22 show changes in volume growth and anatomical results of tumor tissues of each group after inhibiting C57BL/6J tumor-bearing mice transduced with human TIM3 gene by P26-12-peptide (targeting TIM3) for 21 days.
FIG. 23 shows results of tumor weight of the tumor tissues in each experimental group after inhibiting C57BL/6J tumor-bearing mice transduced with human TIM3 gene by P26-12-peptide (targeting TIM3) for 21 days.

### DETAILED DESCRIPTION

In order to better understanding of the implementation strategies and embodiments of the present disclosure, examples are set forth and exemplified below. In the case that the experimental procedures in the following examples are not specifically described, they are generally operated according to conventional conditions or manufacturer's (company's) instructions. The following examples are merely illustrative of the practice of the invention and are not intended to limit the scope of the invention.

**The main reagents and instruments used in the invention:** Ph.D.-12/20 phage display peptide library kit (New England Biolabs, USA); Human lymphocyte isolation solution Histopaque-1077 (Cat # 10771-6×100ML, Sigma Aldrich); CD14+ Microbeads (Cat # 130-050-201), CD4+T cell separation kit (Cat# 130-096-533), Mo-DC Generation Toolbox I (Cat # 130-093-568), MidiMACS separator (Cat # 130-042-302), LS column (Cat # 130-042-401) purchased from Miltenyi Biotech; Human IFN-γ DuoSet ELISA (DY285B) and Human IL-2 DuoSet ELISA (DY202) purchased from R&D Systems; TMB substrate reaction solution (D0022, Suzhou Yako Technology Co., Ltd.); PD-1 inhibitor Opdivo (NDC 0003-3772-11, Bristol-Myers Squibb); Galectin-9 protein (cat#CHI-HF-210GAL9) and TIM3 protein (cat#CHI-MF-210T3-C050) purchased from Chimerigen; anti-TIM3 monoclonal antibody C23 produced by the inventors' laboratory; Anti-GAPDH (10494-1-AP, Proteintech) anti-HRP-murine secondary antibody (ab6789, Abcam).

**Cell lines and culture:** Human renal epithelial cell lines 293T and 293T-TIM3 cell lines (KYinno co., Ltd. Beijing, China); RPMI-1640 medium (#11875500BT), DMDM (#SH30243.01) and fetal bovine serum (FBS; # SV30087.02, Gibco, USA); Penicillin/streptomycin solution (SV30082.01) purchased from Beijing Sunshine Biotechnology Co., Ltd.; Mo-DC differentiation medium (130-094-812), and Mo-DC maturation medium (130-094-813) purchased from Miltenyi Biotec; Each medium was supplemented with 10% fetal bovine serum (FCS) and 1% penicillin/streptomycin and incubated with 5% CO₂ at 37°C.

### Example 1: Phage Random Peptide Library Technique Screening to Obtain Binding Peptides Targeting TIM3

### Analysis and Identification of TIM3 Expression in 293T-TIM3 Cell Lines by Western blot

293T-TIM3 cells are tool cells used in the application to screen random phage display libraries and to identify specific affinities of TIM3-targeting binding peptides. To verify the expression of TIM3 in 293T cells, these cells were identified by Western blotting. 293T/293T-TIM3 cells were lysed with lysis buffer (1× protease inhibitor mixture from Roche, 2 mM EDTA, 2mM dithiothreitol, 150 mM NaCl, 50 mM Tris-HCl, pH 7.0 and 1%SDS). The protein concentration used in the experiment was qualitatively determined by BCA protein detection kit (Thermo Fisher Scientific, USA). After gel electrophoresis and Western blotting analysis of cell lysate, the protein bands were identified qualitatively and quantitatively by Image Pro Plus 6.0 software. After lysis of the negative cells (293T) and the positive cells (293T-TIM3), two loading systems (total protein amounts of 30 µg and 15 µg, respectively) of the extracted total proteins were detected, and the TIM3 protein band at 55kDa was detected. The contents of TIM3 protein in both the negative cells (293T) and the positive cells (293T-TIM3) were qualitatively and quantitatively detected, and TIM3 was not detected in the negative cells (293T), but detected in the positive cells (293T-TIM3). The results met the requirements of the experiment.

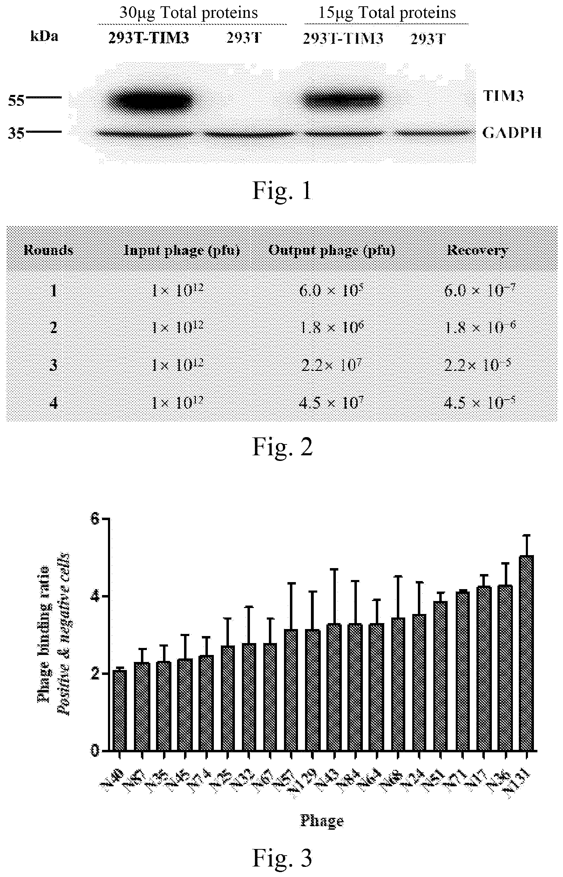

FIG. 1 shows Western Blot detection to identify stable expression of recombinant human TIM3 in 293T cells.

### Escherichia coli ER2738 and cell culture

293T and 293T-TIM3 cells were incubated with DMEM containing 10%FBS in a suitable environment (37°C, 5% CO₂), and then blocked with 1%BSA-PBS for 1 h prior to screening. *Escherichia coli* ER2738 was plated overnight on Luria-Bertani-tetracycline (LB-Tet) at 37°C, and the cultures were then transferred to LB medium to achieve a logarithmic growth phase for use.

### Amplification and Subtraction Screening of Phage Display Peptide Library

Phages (10 µL, 10¹¹pfu) were added to 293T and 293T-TIM3 cells (1×10^{6~7}) at 80% confluence for screening. That is, after the phage fluid first bind to the negative adherent 293T cells, the phages not bound to the 293T cells were transferred and incubated with the 293T-TIM3 target cells under the binding condition of 4°C and a 360° shaker for 1 hour. Finally, eluted primary peptide library was obtained by washing. The first screening was washed with PBS, after which the buffer used for washing was Tween-20 (PBST) increased from 0.05% (v/v) and 0.1% (v/v) to 0.5% (v/v). Subsequently, the phages bound to the surface of the target cell 293T-TIM3 was titrated and expanded in *E. coli* ER2738 culture. The recovered phages were repeatedly performed for the next round of *in vitro* screening (a total of four rounds of screening experiments were performed).

### Detailed workflow

### Panning

### Day 1: preparation

1. Pre-block of EP tube: 1% BSA was added to a 5mL EP tube and incubated overnight at 4°C on a 360 ° shaker.
2. Pre-preparation of 293T and 293T-TIM3 cells: 10⁶-10⁷ cells/plate were incubated overnight in a 37°C incubator.

### Day 2 (panning 1)

1. Single clones were picked from ER2738 plates, and 20 mL of bacteria were shaken in advance, and shaken vigorously for culture on a shaker at 37°C for 4 h until early logarithmic growth (OD600=0.5).
2. Preparation of single cell suspension: Overnight cultured cells were centrifuged successively, and the pellets were resuspended in 2mL PBS (2×10⁷ cell) and placed in 5mL EP tubes.
3. Adsorption binding: 10µL peptide library (Input1) was first added to an EP tube of 293T cells, and incubated on a 360° shaker at 4°C for 1 h. The culture was then centrifuged at 640×g for 2min, and the supernatant was transferred to an EP tube of 293T-TIM3 cells, and incubated on a 360° shaker at 4°C for 2 h, and then centrifuged at 640×g for 2min. The pellets were washed 5 times with 10mL PBS for 5 min each time.
4. Infection expansion: The cell pellets were resuspended in LB medium, and 20 mL of ER2738 bacterial solution at early logarithmic phase (OD=0.5) was added and shaken vigorously for culture on a shaker at 37°C for 4.5h (a conical culture dish of 250 mL or more, 225/rpm). The culture was centrifuged at 4°C/10Krpm for 10 min.
5. The supernatant portion was taken out, and 1/6 volume of 2.5M PEG/NaCl was added to the supernatant portion and precipitated overnight at 4°C.
6. Pre-block of EP tubes: 1% BSA was added to a 5mL EP tube and incubated overnight on a 360° shaker at 4°C.
7. Pre-preparation of 293T and 293T-TIM3 cells: 10⁶-10⁷cells/plate was incubated overnight in an incubator at 37°C.

### Day 3 (panning 2)

1. Single clones were picked from ER2738 plates, and 20 mL of bacteria were shaken in advance, and shaken vigorously for culture on a shaker at 37°C for 4 h until early logarithmic growth (OD600=0.5).
2. The supernatant after infection expansion yesterday was recovered and centrifuged at 4°C/12Krpm for 15 min. The supernatant was discarded, and the pellets were resuspended in PBS to a volume of 1.5mL and centrifuged at 4°C/12Krpm for another 5 min. The residual pellets were discarded. The supernatant was transferred and incubated with 1/6 volume of 2.5M PEG/NaCl at 4°C for 60 min. After centrifugation at 4°C/12K rpm for 10 min, the supernatant was discarded and the pellets were resuspended in PBS to a volume of 200µL and stored at 4°C (the first round of secondary peptide library).
3. Preparation of single cell suspension: Overnight cultured cells were centrifuged successively, and the pellets were resuspended in 2mL PBS (2×10⁷ cell) in a 5mL EP tube.
4. Adsorption binding: 100 µL of the first round of secondary peptide library (Input2) was first added to an EP tube containing 293T cells, incubated on a 360° shaker at 4°C for 1 h, and then centrifuged at 640×g for 2min. The supernatant was transferred to an EP tube containing 293T-TIM3 cells, incubated on a 360° shaker at 4°C for 1 h, and centrifuged at 640×g for 2min. The pellets were washed 10 times with 10mL 0.05% PBS-T for 5 min each time.
5. Infection expansion: The cell pellets were resuspended in LB medium, and 20 mL of ER2738 bacterial solution at early logarithmic phase (OD=0.5) was added and shaken vigorously for culture on a shaker at 37°C for 4.5h (a conical culture dish of 250 mL or more, 225/rpm). The culture was centrifuged at 4°C/10Krpm for 10 min.
6. The supernatant portion was taken out, and 1/6 volume of 2.5M PEG/NaCl was added to the supernatant portion and precipitated overnight at 4°C.
7. Pre-block of EP tubes: 1% BSA was added to a 5mL EP tube and incubated overnight on a 360° shaker at 4°C.
8. Pre-preparation of 293T and 293T-TIM3 cells: 10⁶-10⁷cells/plate was incubated overnight in an incubator at 37°C.

### Day 4 (panning 3)

1. Single clones were picked from ER2738 plates, and 20 mL of bacteria were shaken in advance, and shaken vigorously for culture on a shaker at 37°C for 4 h until early logarithmic growth (OD600=0.5).
2. The supernatant after infection expansion yesterday was recovered and centrifuged at 4°C/12Krpm for 15 min. The supernatant was discarded, and the pellets were resuspended in PBS to a volume of 1.5mL and centrifuged at 4°C/12Krpm for another 5 min. The residual pellets were discarded. The supernatant was transferred and incubated with 1/6 volume of 2.5M PEG/NaCl in ice water at 4°C for 60 min. After centrifugation at 4°C/12K rpm for 10 min, the supernatant was discarded and the pellets were resuspended in PBS to a volume of 200µL and stored at 4°C (the second round of secondary peptide library).
3. Preparation of single cell suspension: Overnight cultured cells were centrifuged successively, and the pellets were resuspended in 2mL PBS (2×10⁷ cell) in a 5mL EP tube.
4. Adsorption binding: 100 µL of the first round of secondary peptide library (Input3) was first added to an EP tube containing 293T cells, incubated on a 360° shaker at 4°C for 1 h, and then centrifuged at 640×g for 2min. The supernatant was transferred to an EP tube containing 293T-TIM3 cells, incubated on a 360° shaker at 4°C for 1 h, and centrifuged at 640×g for 2min. The pellets were washed 12 times with 10mL 0.05% PBS-T for 5 min each time.
5. Infection expansion: The cell pellets were resuspended in LB medium, and 20 mL of ER2738 bacterial solution at early logarithmic phase (OD=0.5) was added and shaken vigorously for culture on a shaker at 37°C for 4.5h (a conical culture dish of 250 mL or more, 225/rpm). The culture was centrifuged at 4°C/10Krpm for 10 min.
6. The supernatant portion was taken out, and 1/6 volume of 2.5M PEG/NaCl was added to the supernatant portion and precipitated overnight at 4°C.
7. Pre-block of EP tubes: 1% BSA was added to a 5mL EP tube and incubated overnight on a 360° shaker at 4°C.
8. Pre-preparation of 293T and 293T-TIM3 cells: 10⁶-10⁷cells/plate was incubated overnight in an incubator at 37°C.

### Day 5 (panning 4)

1. Single clones were picked from ER2738 plates, and 20 mL of bacteria were shaken in advance, and shaken vigorously for culture on a shaker at 37°C for 4 h until early logarithmic growth (OD600=0.5).
2. The supernatant after infection expansion yesterday was recovered and centrifuged at 4°C/12Krpm for 15 min. The supernatant was discarded, and the pellets were resuspended in PBS to a volume of 1.5mL and centrifuged at 4°C/12Krpm for another 5 min. The residual pellets were discarded. The supernatant was transferred and incubated with 1/6 volume of 2.5M PEG/NaCl in ice water at 4°C for 60 min. After centrifugation at 4°C/12K rpm for 10 min, the supernatant was discarded and the pellets were resuspended in PBS to a volume of 200µL and stored at 4°C (the third round of secondary peptide library).
3. Preparation of single cell suspension: Overnight cultured cells were centrifuged successively, and the pellets were resuspended in 2mL PBS (2×10⁷ cell) in a 5mL EP tube.
4. Adsorption binding: 100 µL of the first round of secondary peptide library (Input4) was first added to an EP tube containing 293T cells, incubated on a 360° shaker at 4°C for 1 h, and then centrifuged at 640×g for 2min. The supernatant was transferred to an EP tube containing 293T-TIM3 cells, incubated on a 360° shaker at 4°C for 1 h, and centrifuged at 640×g for 2min. The pellets were washed 10 times with 10mL 0.05% PBS-T for 5 min each time.
5. Infection expansion: The cell pellets were resuspended in LB medium, and 20 mL of ER2738 bacterial solution at early logarithmic phase (OD=0.5) was added and shaken vigorously for culture on a shaker at 37°C for 4.5h (a conical culture dish of 250 mL or more, 225/rpm). The culture was centrifuged at 4°C/10Krpm for 10 min.
6. The supernatant portion was taken out, and 1/6 volume of 2.5M PEG/NaCl was added to the supernatant portion and precipitated overnight at 4°C.

### Day 6 (titering)

1. The supernatant after infection expansion yesterday was recovered and centrifuged at 4°C/12Krpm for 15 min. The supernatant was discarded, and the pellets were resuspended in PBS to a volume of 1.5mL and centrifuged at 4°C/12Krpm for another 5 min. The residual pellets were discarded. The supernatant was transferred and incubated with 1/6 volume of 2.5M PEG/NaCl in ice water at 4°C for 60 min. After centrifugation at 4°C/12K rpm for 10 min, the supernatant was discarded and the pellets were resuspended in PBS to a volume of 200µL and stored at 4°C (the fourth round of secondary peptide library- input5/output).
2. Titering: 1µL of input1-5 were respectively titrated after 10⁻⁶/⁻⁸/⁻¹⁰ dilutions.

### Screening and expansion of positive clones

Positive clones (blue plaques on the selection plate) were randomly selected after four rounds of subtraction screening. These selected phage plaques were then propagated in *E. coli* ER2738 and stored in 60% sterile glycerol at 20°C.

### Screening and identification of positive phage clones by next generation sequencing (NGS) and enzyme-linked immunosorbent assay (ELISA)

After four rounds of biological screening, 4-grade phage library subjected to screening expansion was divided into two parts, in which a portion of the phage library upon 4-round screening was subjected to next generation sequencing (NGS) (which was completed by a company), a peptide sequence occurred at a higher frequency was preferred; and another portion of the 4-grade phage library was expanded and subjected to cell ELISA to identify positive phage clones that were preferably excellent in bound to 293T-TIM3 cells. That is, supernatants containing phage particles were collected, and differences in the binding capacity of phages to 293T-TIM3 cells and to negative 293T cells were compared (i.e., differences in the binding capacity of phages to negative cells and to positive cells, the ratio of the two OD values is >2) according to the instructions of the 12-mer peptide library kit (phage ELISA using 293T-TIM3 and normal 293T cells). Finally, polypeptides targeting TIM3 were preferably selected based on the experimental results of both NGS and phage ELISA.

FIG. 2 shows phage-positive clones bound to TIM3, which were enriched and recovered by phage 12 peptide library subtraction screening in each round. After 4 rounds of subtraction screening, the number of phages recovered from 293T-TIM3 cells increased by a factor of 75 (6.0×10⁻⁷ of recovery from the first round to 4.5 ×10⁻⁵ of recovery from the fourth round).

FIG. 3 shows cell ELISA comparison and analysis of the ratio of OD490 values of positive clones bound to target cells (293T-TIM3) to those bound to control cells (293T). That is, the OD490 values of 20 preferred positive clones bound to target cells (293T-TIM3) were compared to the OD490 values of the positive clones bound to control cells (293T), showing that the ratios of the OD490 values of positive clones bound to positive/negative cells were greater than 2.

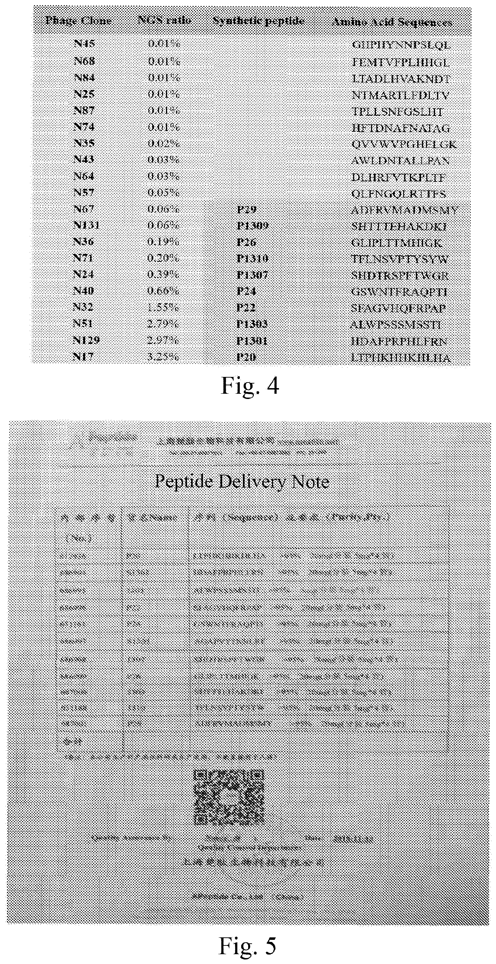

FIG. 4 shows the 12-peptides (targeting TIM3) occurred at a higher frequency via NGS sequencing and their amino acid sequences. That is, the occurrence frequency for the preferred 20 positive phage clones above were identified by high-throughput sequencing with the lowest occurrence frequency of 0.01% and the highest occurrence frequency of 3.25%.

### Sequencing of positive clones and synthesis and labeling of polypeptides

10 12-peptides and 6 20-peptides were preferably selected according to cell ELISA (the ratio of binding values to positive/negative cells > 2) and NGS sequencing (occurrence frequency >0.6%) and were subjected to sequencing synthesis (Shanghai Chu Peptide Biotechnology Co., Ltd.). The peptides were labelled by FITC (Shanghai Qiangyao Biotechnology Co., Ltd.). Unrelated peptides were designed and synthesized as negative controls.

FIG. 5 shows a synthesis report of 10 preferred 12-peptides (targeting TIM3).

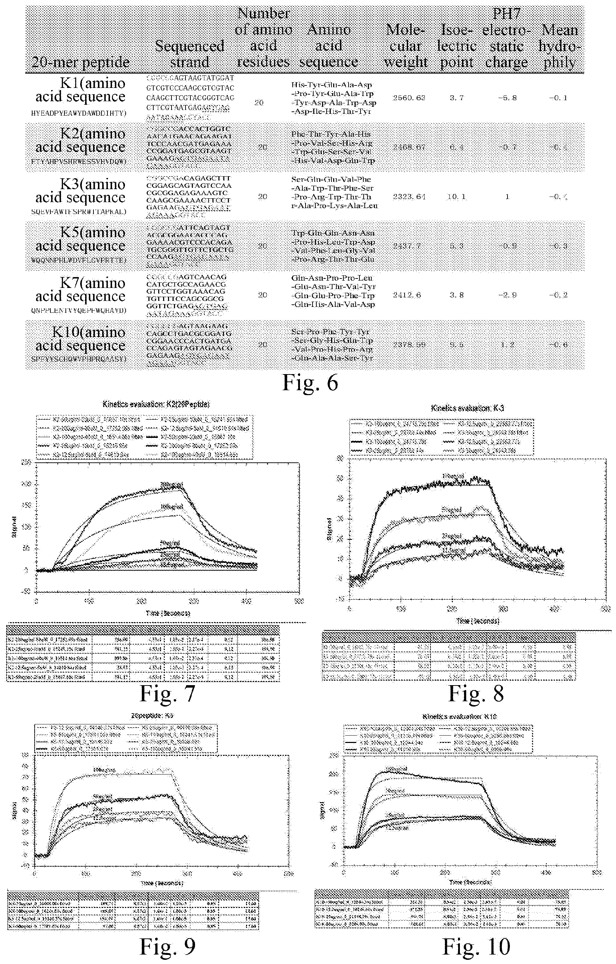

FIG. 6 shows the amino acid sequences and related physicochemical properties of 6 preferred 20-peptides (targeting TIM3).

### Example 2: Affinity Identification of TIM3-Targeting Binding Peptides (Including Identification of Ability to Competitively Bind to Ligand)

The binding properties of the TIM3-targeting binding peptides to TIM3 and their competition experiments with Galectin-9-bound TIM3 were identified by flow cytometry and surface plasmon resonance (SPROpenSPR, Nicoya, V3.01) detection.

**SPR assay:** Affinity identification between the TIM3-targeting binding peptides and TIM3 was performed by immobilizing TIM3 protein (50 µg/mL) on the surface of a nanogold sensor chip upon activation of EDC/NHS. The TIM3-targeting binding peptides were diluted with buffer (PBS, pH 7.4) to concentrations of 9.75µM, 19.5µM, 39µM and 78µM, respectively. The competitive binding ability of the TIM3-targeting binding peptides to the ligand was identified by immobilizing the Galectin-9 protein (25 µg/mL) on a sensor chip at a detection concentration of 100 µg/mL for the TIM3-targeting binding peptides, and at a concentration of the mixed sample of 100 µg/mL TIM3-targeting binding peptides and 10 µg/mL TIM3 protein. The detection sample was loaded at a flow rate of 20µL/min for about 7 min according to the instrument instructions. The KD, ka and kd values were calculated and analyzed by Trace Drawer Evaluation (version 2.0) software.

FIG. 7 shows the affinity (comprising KD values) of K2-20-peptide (targeting TIM3) to the TIM3 protein via SPR detection. The detection concentrations were 12.5 µg/mL, 25 µg/mL, 50 µg/mL, 100 µg/mL and 200 µg/mL, the KD value was 2.27×10⁻⁴ M, ka was 4.53×10⁻¹ Ms⁻¹, and Kd was 1.03×10⁻² S⁻¹.

FIG. 8 shows the affinity (comprising KD values) of K3-20-peptide (targeting TIM3) to the TIM3 protein via SPR detection. The detection concentrations were 12.5 µg/mL, 25 µg/mL, 50 µg/mL and 100 µg/mL, the KD value was 2.01×10⁻⁵ M, ka was 6.54×10⁻² Ms⁻¹, and Kd was 1.32×10⁻² S⁻¹.

FIG. 9 shows the affinity (comprising KD values) of K5-20-peptide (targeting TIM3) to the TIM3 protein via SPR detection. The detection concentrations were 12.5 µg/mL, 25 µg/mL, 50 µg/mL and 100 µg/mL, the KD value was 1.80×10⁻⁵ M, ka was 8.87×10⁻² Ms⁻¹, and Kd was 1.60×10⁻² S⁻¹.

FIG. 10 shows the affinity (comprising KD values) of K10-20-peptide (targeting TIM3) to the TIM3 protein via SPR detection. The detection concentrations were 12.5 µg/mL, 25 µg/mL, 50 µg/mL and 100 µg/mL, the KD value was 2.61×10⁻⁵ M, ka was 8.84×10⁻² Ms⁻¹, and Kd was 2.30×10⁻² S⁻¹.

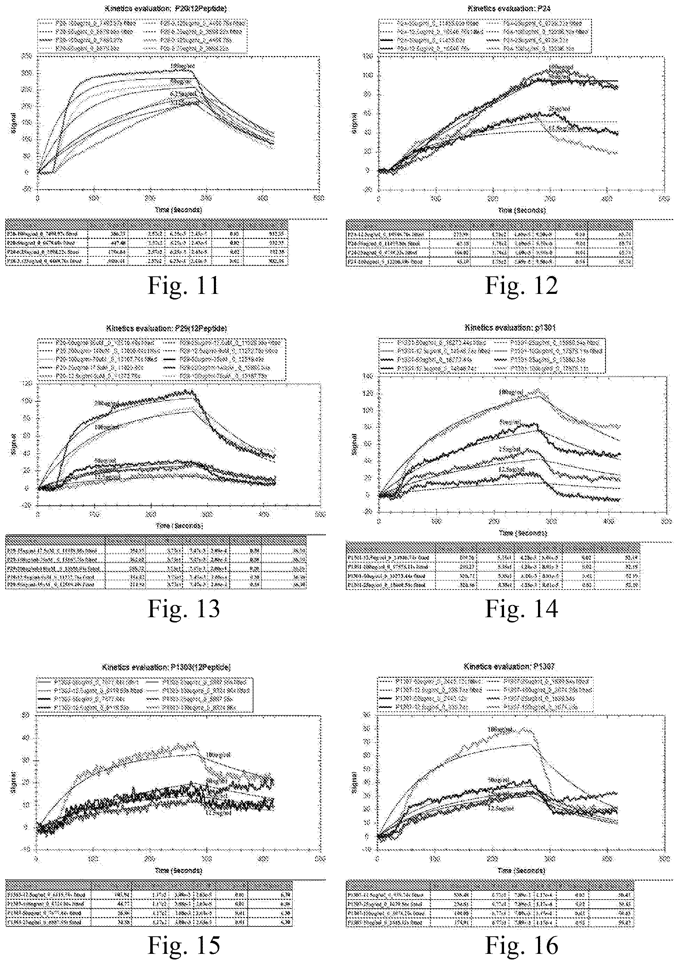

FIG. 11 shows the affinity (comprising KD values) of P20-12-peptide (targeting TIM3) to the TIM3 protein via SPR detection. The detection concentrations were 3.125 µg/mL, 6.25 µg/mL, 50 µg/mL and 100 µg/mL, the KD value was 2.43×10⁻⁵ M, ka was 2.57×10⁻² MS, and Kd was 6.25×10⁻³ S⁻¹.

FIG. 12 shows the affinity (comprising KD values) of P24-12-peptide (targeting TIM3) to the TIM3 protein via SPR detection. The detection concentrations were 12.5 µg/mL, 25 µg/mL, 50 µg/mL and 100 µg/mL, the KD value was 9.50×10⁻⁸ M, ka was 1.78×10⁻² Ms⁻¹, and Kd was 1.69×10⁻⁵ S⁻¹.

FIG. 13 shows the affinity (comprising KD values) of P29-12-peptide (targeting TIM3) to the TIM3 protein via SPR detection. The detection concentrations were 12.5 µg/mL, 25 µg/mL, 50 µg/mL, 100 µg/mL and 200 µg/mL, the KD value was 2×10⁻⁴M, ka was 3.73×10⁻¹ Ms⁻¹, and Kd was 7.47×10⁻³ S⁻¹.

FIG. 14 shows the affinity (comprising KD values) of P1301-12-peptide (targeting TIM3) to the TIM3 protein via SPR detection. The detection concentrations were 12.5 µg/mL, 25 µg/mL, 50 µg/mL and 100 µg/mL, the KD value was 8.01×10⁻⁵M, ka was 5.35×10⁻¹ Ms⁻¹, and Kd was 4.28×10⁻³ S⁻¹.

FIG. 15 shows the affinity (comprising KD values) of P1303-12-peptide (targeting TIM3) to the TIM3 protein via SPR detection. The detection concentrations were 12.5 µg/mL, 25 µg/mL, 50 µg/mL and 100µg/mL, the KD value was 2.63×10⁻⁵M, ka was 1.17×10⁻² Ms⁻¹, and Kd was 3.08×10⁻³ S⁻¹.

FIG. 16 shows the affinity (comprising KD values) of P1307 (targeting TIM3) to the TIM3 protein via SPR detection. The detection concentrations were 12.5 µg/mL, 25 µg/mL, 50 µg/mL and 100 µg/mL, the KD value was 1.17×10⁻⁴M, ka was 6.77×10⁻¹ Ms⁻¹, and Kd was 7.89×10⁻³S⁻¹.

**Flow cytometry assay**: 293T-TIM3 and 293T cells (negative control cells) were washed with PBS buffer and counted, and then the cells were blocked with 1%BSA-PBS for 30 minutes at room temperature, and washed with PBS buffer and centrifuged for use. The cells were then resuspended in PBS, and transferred to EP tubes subjected to sterilization under high pressure, each of which contained 500µl cell suspension (2×10⁶ cells). The synthetic TIM3-targeting binding peptides coupled with FITC were added to the cell suspension with final concentrations of 50 µg/mL, 16.7 µg/mL and 5.6 µg/mL. The final concentration of Galectin-9 for competitive inhibition was 10 µg/mL. After the related peptides, reagents and cells were incubated at room temperature for 30 minutes and washed twice with PBS, the cells were resuspended in 200 µL PBS for flow cytometry analysis. The experimental results were analyzed by FlowJo software.

### Example 3: Identification of in vitro Functional Activity of TIM3-targeting Binding Peptides

Identification of the functional activity of TIM3-targeting binding peptides was performed by evaluating the levels of activation and proliferation of T cells, i.e., the levels of expression of IL-2 and IFN-γ cytokines. First, Dendritic Cells (DCs) and CD4+T cells were obtained by magnetic bead sorting, and then the levels of IFN-γ cytokines and IL-2 were detected by a Mixed Lymphocyte Reaction (MLR) experiment simulating close to human physiological environment and an ELISA experiment, to identify biological activities of the TIM3-targeting binding peptides.

### Isolation and Purification of Dendritic Cells (DC) and CD4 + T Cells

1. Blood dispensing: 20 mL of whole blood from healthy subjects was cooled to room temperature (25°C), and mixed with normal saline or 1×PBS to the final volume of about 36mL, and inverted thoroughly. The cell separation solution was added to a 15mL centrifuge tube at an amount of 5mL/tube for total 4 tubes. The whole blood mixture was dispensed into the centrifuge tubes containing lymphocyte separation solution with an amount of approximately 10 mL per tube.
2. Centrifugation: The centrifuge tubes were centrifuged at 1500 rpm (centrifugal force of 400g) at room temperature for 40 min to separate cell layers. (Supernatant - mononuclear cell layer - separation fluid - red blood cells)
3. Collection of mononuclear cell layer: PBMC buffy coat was aspirated out using a fine pipette (lymphocytes and monocytes, etc.) and an equal volume of PBS was added.
4. Washing cells with PBS and counting: The collected buffy coat cells were adjusted to the volume of 40-45 mL with PBS and centrifuged at 1500rpm for 10 min. The supernatant was aspirated and discarded, and washing and lysis were continued as appropriate (The supernatant was aspirated and discarded after centrifugation. Note that cells should not be aspirated and a small amount of liquid should be retained at the bottom of the tube. 2 mL of erythrocyte lysis buffer was added and lysed on ice for 5-10min. After lysis, the tubes were adjusted to the volume of 40-45 mL with PBS and centrifuged at 1000 rpm for 10 min. The supernatant was aspirated and decanted, and the cell pellets were resuspended in 5mL buffer. PBMCs were counted, and were centrifuged at 1200 rpm for another10 min. If there are few red blood cells, they cannot be lysed).
5. Incubation on ice: The supernatant was discarded, and pre-cooled buffer (80 µL/10⁷cells) (the buffer for incubation was preserved on ice, and the buffer for passing a column was preserved at room temperature) and CD14+ (20µL/10⁷cells) were added according to the number of cells and incubated on ice for 10 min.
6. Preparation of magnetic bead sorting: At the time of centrifugation, a stage and a filter were set up, and two magnetic bead filters and push tubes, four centrifuge tubes, and eluent buffer were prepared. After centrifugation, the supernatant was discarded, and the pellets were resuspended in 500 µL Buffer (500µL/10⁸ cells) and pipetted up and down evenly without bubbles as possible as can.
7. Passing-column: 3mL buffer was added to the column for washing. Then, a bubble-free cell mixture pipetted up and down evenly which was subjected to incubation on ice was added to the filter column, and after that, the column was washed three times with buffer at 3mL each time, while the liquid in the column was not allowed to dry.
8. Cell collection: The LS separation column was removed from the base along with the separator. 3mL eluent was added and the elution fractions were collected, which was repeated twice. The in-column binding fraction, i.e., the pushed-off elution fraction, is DC cell. 10 µl of unbound fractions were counted for being used to isolate CD4+ T cells. DC cells and cells to be isolated were centrifuged at 1500 rpm for 10 min.
10. DC was added to the specialized culture medium and incubated in an incubator. The supernatant was discarded, and the pellets were resuspended in DC cell differentiation medium (10mL/10⁷ cells) (RMPI 1640 + 10%FCS, IL-4 20ng/mL, GM-CSF 50 ng/mL) and incubated in a 25 mL culture flask at 37°C. The medium was supplemented on day 3 and replaced on day 7, and cells were collected on day 10.
11. CD4+ T cell isolation:
   1) Counting and centrifugation: Cell suspension rich in CD4+T cell was counted and centrifugated at1500rpm at 4°C for10 min.
   2) Incubation: The supernatant was discarded. The cell pellets were resuspended in a pre-cooled buffer (40 µL/10⁷ cells), to which a biotin-labeled primary antibody (10 µL/10⁷ cells) was then added and mixed, and incubated at 4°C or on ice for 5 min. Then the pre-cooled buffer (30 µL/10⁷ cells) and magnetic bead secondary antibody against biotin (20 µL/10⁷ cells) were added and mixed, and incubated at 4°C or on ice for 15 min.
   3) Washing and centrifugation: 10 mL buffer was added and centrifuged at 1500rpm at room temperature for 10 min. The supernatant was discarded and the cell pellets were resuspended in 500 µL buffer (500 µL/10⁸).
   4) Filtering: A LS separation column was loaded and clamped in a separator attached to a base, and 3mL eluent was added to rinse once. 500 µL cell suspension was added to the LS column and 3 mL eluent was added for three times. Unbound fractions were collected.
   5) Collection of CD4+ T cells: The unbound portions of the flow of the collection column are CD4+ T cells. The unbound portions were centrifuged. The supernatant was discarded, and the cell pellets were resuspended in basal medium (RMPI 1640 + 10%FCS) for immediate use.

### Mixed Lymphocyte Reaction (MLR)

Culture of Dendritic cells (DCs): Isolated dendritic cells (DCs) were cultured in Mo-DC differentiation medium (10⁷ cells/10 mL medium), and 10 mL of the same medium was added to the cells after 3 days (the cells were cultured in the differentiation medium at 37°C, 5% CO2 and > 95% humidity for 6 days). On day 7, DCs were resuspended in Mo-DC mature medium and cultured for further 3 days by addition of the Mo-DC differentiation medium. Matured DC and newly isolated T cells (DC and T cells from different donors) were mixed for mixed lymphocyte reaction. Approximately 1×10⁴ DCs and 1×10⁵ T cells per well were mixed and cultured in 200 µL RPMI-1640 medium with 10%FBS. To evaluate the activity (gradient action), competitive inhibition (P26 and ligand), synergistic effect (TIM3-targeting binding peptides and the PD-1 inhibitor Opdivo) of the binding peptides TIM3-targeting binding peptides, the corresponding concentrations of peptides, Opdivo, Galectin-9 were added to the corresponding wells to interact with the cells. The expression levels of IFN-γ (or IL-2) secreted in the cell supernatant were measured on day 5.

### Function Identification of Activating T Cell of TIM3-Targeting Binding Peptides (ELISA Assay)

T cell activity were tested according to the instructions of the ELISA kit. Briefly, the capture antibody dilutions were coated on a 96-well plate overnight (24 hours) at room temperature at a concentration of 2 µg/mL in a volume of 100 µL. On the following day, the capture antibody solution was aspirated and the wells were washed three times with 300 µL wash buffer PBST (0.05%Tween^{®}20, pH 7.2-7.4). Thereafter, the test wells were blocked with 300 µL of 1%BSA-PBS for 1 hour. After blocking, the test wells were washed in the same manner as above and 100 µL of the test samples or standards (in 1%BSA-PBS) were pipetted into each well for 2 hours. Thereafter, the detection antibody (125 ng/mL, 100 µL) was added to each well according to the instructions of the human IFN-γ/IL-2 Duo Set ELISA kit, and incubated for 2 hours. Then, HRP (100 µL, incubated for 20 minutes) and TMB (100 µL) were added. At the end of each step, each well was washed in the same manner as described above. After completion of the color development, the test wells were terminated with 12.5% H₂SO₄ (50 µL/well) and read on an ELISA plate reader at a wavelength of 450/570 nm. All experimental test wells were repeated.

### Detailed experimental procedure

### Reagent Formulation

1. Wash Buffer: PBST; That is, 0.05%Tween-20 was formulated with 1×PBS (PH7.2-7.4) (500mLPBS+250 µl Tween20).
2. Block Buffer: 1%BSA; That is, BSA was dissolved in PBS (pH 7.2-7.4) to a final concentration of 1% and filtered through a 0.2 µm filter (0.5 g BSA in 50 mL PBS).
3. Reagent Diluent: see the above-mentioned Block Buffer.
4. Horseradish peroxidase labeled streptavidin (Streptavidin-HRP) was provided as a stock solution in a kit and stored at 4°C (diluted with a diluent, i.e., a block buffer, at 1:40).
5. Preparation of stock solutions of murine anti-human IFN-γ and IL-2 coated antibody (Capture): The lyophilized antibody to be used in the kit was first centrifuged (120 µg of the dry powder of anti-human IFN-γ antibody and 240 µg of the dry powder of anti-IL-2 coated antibody in each container were dissolved in 0.5 mL 1×PBS to prepare stock solutions (240 µg/mL, 480 µg/mL) so that the final concentrations of anti-INF-γ and anti-IL-2 coated antibody were 2µg/mL and 4µg/mL, respectively, and were diluted at 1: 120 in use.
6. Preparation of stock solutions of biotinylated goat anti-human IFN-γ and IL-2 detection antibody: The lyophilized biotinylated goat anti-human IFN-γ antibody and the lyophilized IL-2 detection antibody to be used in the kit were first centrifuged (the biotinylated anti-human IFN-γ antibody and the IL-2 detection antibody in each container were respectively 7.5 µg and 6 µg), and dissolved in 1 mL diluent, i.e., the block buffer 1%BSA to respectively prepare 7500 ng/mL and 6000 ng/mL of stock solutions, which were used in the final concentrations of 125 ng/mL and 100 ng/mL. Therefore, the stock solutions were diluted at 1: 60 in use.
7. Standards: IFN-γ (95 ng/vial) and IL-2 (30 ng/vial) (stored at 4°C) were dissolved in 0.5 mL of the dilution 1%BSA to the final concentrations of 190 ng/mL for IFN-γ and 60 ng/mL for IL-2. Dilutions were made, as appropriate, depending on the actual conditions of the experiment.
8. Substrate solution: TMB, which was used directly.
9. Reaction termination solution: 12.5% H₂SO₄.

### Experimental Procedure

1. Antibody coating: 120 µg of IFNγ coated antibody dry powder and 240 µg of IL-2 coated antibody dry powder were dissolved by adding 0.5 mL 1× PBS to prepare a stock solution (240 µg/mL and 480 µg/mL), and the final concentrations of the anti-INF-y antibody and the anti-IL-2 antibody were 2 µg/mL and 4 µg/mL, respectively, so that they were diluted at 1: 120 in use. The antibodies were coated overnight at room temperature at 50 µL/well.
2. Blocking: The following day after coating, the plates were blocked with the above-mentioned blockbuffer, 1%BSA, at room temperature for 2 h, and then washed three times with PBST.
3. Addition of culture supernatant and standards: The cell culture supernatant to be tested and the standards were added at 50 µL/well, and incubated at room temperature for 2 h. The plates were washed three times with PBST.
4. Addition of biotinylated anti-human INF-γ detection antibody: The biotinylated goat anti-human INF-γ stock solution (7500 ng/mL) and IL-2 (6000 ng/mL) detection antibody were diluted 1:60 with a diluent, i.e., the block buffer 1%BSA. 50µL of the diluted antibodies were added to each well, and incubated for at room temperature for 2 hours. The plates were washed three times with PBST. (The final concentrations of anti-human INF-γ and IL-2 were 125 ng/mL and 100 ng/mL respectively).
5. HRP-labeled streptavidin: HRP-labeled streptavidin was diluted 1:40 with a diluent, i.e., the block buffer, and were added at 50 µL/well, and incubated at room temperature for 20 min. The plates were washed three times with PBST.
6. Color development: The chromogenic substrate TMB was added at 100 µL/well, and left at room temperature for 5-20min in dark (depending on the actual situation).
7. Termination of color development: 12.5% of thetermination solution H₂SO₄ was added at 50 µL/well.
8. On-machine reading: The plates were read by using a microplate reader at 450nm/570nm.

FIG. 17 shows the identification of the biological activity (INF-γ levels expressed by activated T cells) of the 20-peptides (targeting TIM3) K1, K2, K3, K5, K7, K10 of the MLR system detected by ELISA. As shown in the figure, NC0 contains only T cells, NC is a mixture of DCs and T cells, Con is a mixture of DCs and T cells plus negative control peptide, and experimental groups are DCs and T cells plus K1, K2, K3, K5, K7, K10, respectively. There was a significant difference between experimental groups and control groups (P < 0.01), and the expression level of INF-γ was significantly increased.

FIG. 18 shows the identification of the biological activity (INF-γ levels expressed by activated T cells) of 12-peptides (targeting TIM3) P20, P22, P24, P26, P29, P1301, P1303, P1307, P1309, P1310 of the MLR system detected by ELISA. As shown in the figure, the experimental principle was the same as that in FIG. 17 above. NC0 contains only T cells, NC is a mixture of DCs and T cells, P0 is a mixture of DCs and T cells plus negative control peptide. Some experimental groups, P1310, P1301 (P < 0.01), P29, P1303 and P26 (P < 0.05) were significantly different compared with the control group, and INF-γ expression levels were significantly increased.

FIG. 19 shows the binding affinity of P26-12-peptide (targeting TIM3) to the TIM3 protein, in which Panel A: the KD value of P26 bound to TIM3 detected by SPR (a TIM3 sensor chip); Panel B: the binding curve of the negative control peptide P0 to TIM3 detected by SPR; Panel C: the KD value of Galectin-9 (a ligand of TIM3) bound to TIM3 detected by SPR (a TIM3 sensor chip); Panel D: the experiment of P26 competing with the ligand Galectin-9 for binding to TIM3 detected by SPR (a Galectin-9 sensor chip) (difference in binding signals between TIM3 and Galectin-9 vs. the mixture of TIM3 and P26 and Galectin-9) was detected by SPR; and Panel E: the binding specificity of different concentrations of P26-FITC to 293T-TIM3 cells and the ability of P26-FITC competing with Galectin-9 for binding to 293T-TIM3 cells examined by flow cytometry.

FIG. 20 shows the functional activity experiment of P26-12-peptide (targeting TIM3) and its combinations with other drugs in activation of CD4+T cell (MLR system, ELISA assay). Panels A and B show the levels of IFN-γ and IL-2 secreted by CD4+ T cells activated by different concentrations of P26. Panels C and D illustrate a synergistic experiment of P26 and Opdivo (a PD-1 inhibitor), and show the levels of IFN-γ and IL-2 secreted by CD4+ T cells activated by P26 (100 µg/mL), Opdivo (0.1 µg/mL), the combination of P26 (100µg/mL) and Opdivo (0.1 µg/mL). Panels E and F illustrate a competition inhibition experiment of P26 and the ligand Galectin-9, and show that P26 (100 µg/mL) reversed Galectin-9 (5 µg/mL)-mediated inhibition of CD4+ T cell activation (increased expression levels of IFN-γ and IL-2).

### Example 4: In vivo anti-tumor growth effect of TIM3-targeting binding peptides

In this example, the anti-tumor growth effect of the TIM3-targeting binding peptides was further examined in mice. The tumor inhibitory effect of the TIM3-targeting binding peptides on C57BL/6J MC38-hPDL1 tumor-bearing mice humanized with TIM3 was detected by the following experimental steps:
1. Inoculation of mice: M38-hPDL1 tumor cells (1×10⁶ cells/mouse) were inoculated subcutaneously into C57BL/6J mice transduced with the human TIM3 gene.
2. Grouping: Mice were randomly divided into 4 groups with 7 mice per group when tumors reached 80~100mm³.
3. Administration: PBS (negative control), PD-L1 humanized antibody (positive control), polypeptides alone (experimental group) and polypeptides plus PD-L1 humanized antibody (synergistic effect group) were administered intraperitoneally to mice in each group, respectively. The amount of antibody used was 10 mg/kg twice a week. The amount of polypeptide was 5 mg/kg once daily. Tumor size and body weight of the mice were measured every 3 days.
4. Post-administration treatment: mice were euthanized 3 weeks after administration. Tumors were isolated, weighed, and photographed.

The grouping and administration information are shown in Table 1.

**Table 1**

| **Grouping and administration information** | | | | | | | |
|---|---|---|---|---|---|---|---|
| **Group** | **Animal number** | **Treatment** | **Administration dose (mg/kg)** | **Route of administration** | **Dose µL/g** | **Administration period** | **Total amount required (mg)** |
| G1 | 7 | Vehicle | - | intraperitoneal injection | 10 | twice a week for three weeks | 0 |
| G2 | 7 | ATE | 10 | intraperitoneal injection | 10 | twice a week, for three weeks | 10 mg |
| G3 | 7 | P26 | 5 | intraperitoneal injection | 10 | QD (once a day) | 17 mg |
| G4 | 7 | P26+ATE | 5 + 10 | intraperitoneal injection | | | P26 17 mg; ATE 10 mg |

| | | | | | | | |
|---|---|---|---|---|---|---|---|
| Vehicle: blank control group; ATE: PD-L1 inhibitor group. | | | | | | | |

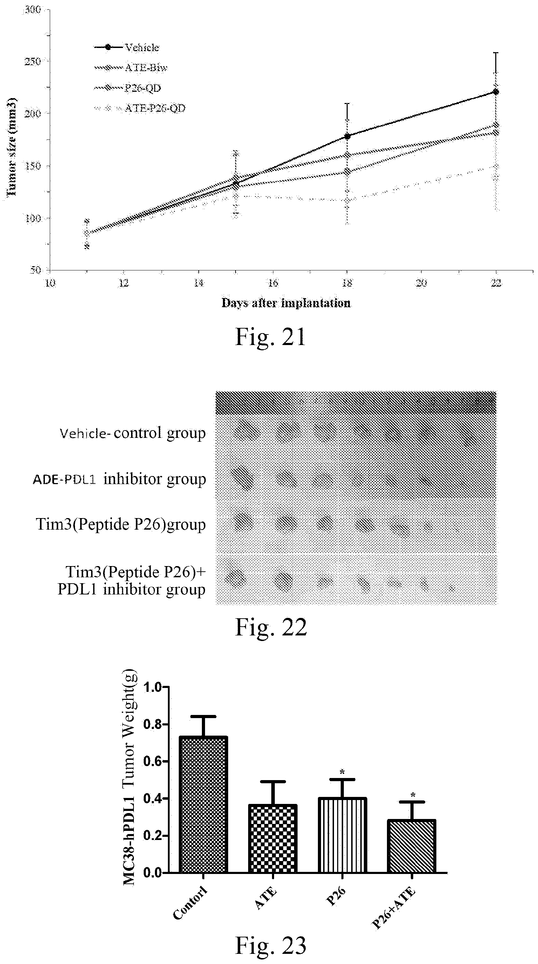

FIGs. 21 and 22 show changes in volume growth and anatomical results of tumor tissues of each group after C57BL/6J tumor-bearing mice transduced with the human TIM3 gene were inhibited by P26-12-peptide (targeting TIM3) for 21 days. As seen from the experimental results, the tumor volumes in the peptide group (P26), and the ATE (PD-L1 inhibitor) + peptide group (P26) tended to decrease compared to the control group.

FIG. 23 shows the results of tumor weight of tumor tissues of each experimental group after C57BL/6J tumor-bearing mice transduced with the human TIM3 gene were inhibited by P26-12-peptide (targeting TIM3) for 21 days. As seen from the experimental results, the growth of the tumor was inhibited in the peptide group (P26) and the ATE (PD-L1 inhibitor) + peptide group (P26), which were significantly different compared with the control group (P < 0.05).

## Claims

1. A polypeptide of (a) or (b) or a pharmaceutically acceptable salt thereof:
(a) a polypeptide consisting of the amino acid sequence as set forth in any one of SEQ IDNo.1 to SEQ ID No. 16;
(b) a derived polypeptide which is obtained by substituting, adding and/or deleting one or more amino acids in the amino acid sequence as set forth in any one of SEQ ID No.1 to SEQ ID No. 16 and has the same function as (a).

2. Use of the polypeptide of claim 1 as an immune checkpoint TIM3-targeting binding peptide.

3. A polynucleotide encoding the polypeptide of claim 1.

4. A fusion protein comprising the amino acid sequence of the polypeptide of claim 1.

5. A pharmaceutical composition comprising the polypeptide or a pharmaceutically acceptable salt thereof of claim 1, or the polynucleotide of claim 3, or the fusion protein of claim 4; and a pharmaceutically acceptable carrier or excipient.

6. The pharmaceutical composition according to claim 5, further comprising a PD-1 inhibitor or a PD-L1 inhibitor, wherein the PD-1 inhibitor is preferably Opdivo, and the PD-L1 inhibitor is preferably atezolizum.

7. Use of the polypeptide of claim 1 or the polynucleotide of claim 3 or the fusion protein of claim 4 or the pharmaceutical composition of any one of claims 5 to 6 in the manufacture of a reagent for enhancing T cell activity, wherein the reagent for enhancing T cell activity is preferably used to promote elevated expression of immune factors IFNγ and/or IL-2 secreted by T cells.

8. Use of the polypeptide of claim 1 or the polynucleotide of claim 3 or the fusion protein of claim 4 or the pharmaceutical composition of any one of claims 5 to 6 in the manufacture of a reagent that has the ability to reverse Galectin-9 mediated inhibition of T cell activation.

9. Use of the polypeptide of claim 1 or the polynucleotide of claim 3 or the fusion protein of claim 4 or the pharmaceutical composition of any one of claims 5 to 6 in the manufacture of a medicament for inhibiting tumor growth.

10. Use of the polypeptide of claim 1 or the polynucleotide of claim 3 or the fusion protein of claim 4 or the pharmaceutical composition of any one of claims 5 to 6 in the manufacture of a medicament for the treatment of relevant tumors and/or inflammatory diseases by inhibition of TIM3 signaling.

11. An anti-tumor method comprising administering to a subject an effective amount of the polypeptide of claim 1 or the polynucleotide of claim 3 or the fusion protein of claim 4 or the pharmaceutical composition of any one of claims 5 to 6, wherein the polypeptide of claim 1 is used to bind to TIM3 to inhibit tumor growth.
